# EUROPEAN PATENT APPLICATION

(11) **EP 2 263 552 A1**
(43) Date of publication of application: **22.12.2010**
(21) Application number: 09162923.8
(22) Date of filing: 17.06.2009
(51) Int. Cl.: A61B 10/02, A61B 10/00

(54) **Snap-release cell collection device**

(71) Applicant: Switch bvba, 9890 Gavere (BE)
(72) Inventor: Magniette, Olivier, 9831 Deurle (BE)
(74) Representative: Brants, Johan P.E.

(57) **Abstract**

The invention relates to a cell collection device (1), comprising a means for collecting cells (2) and a longitudinal handle (3), whereby said means for collecting cells (2) is attachable to the longitudinal handle (3) using a snap release joint (4). The invention further relates to means for collecting cells (2), comprising a fortification zone (11) and a shape suitable for being secured to a longitudinal handle (3) to form a snap-release joint (4). The invention also relates to a method of collecting a sample with a cell collection device, comprising the steps of: a) providing a cell collection device (1) comprising a means for collecting cells (2) and a longitudinal handle (3) mutually securable with a snap-release joint (4); and b) applying a pressure force on the snap-release joint (4) suitable to disengage the longitudinal handle from the means for collecting a sample.

## Description

### Field of the invention

The present invention relates to a device for sampling a collection of cells, in particular for obtaining a sample from a body cavity such as the uterus mouth or cervix.

### Background of the invention

Physicians commonly acquire clinical specimens by scraping or brushing a target tissue with a specimen collection device. One such application of this methodology is the Papanicolaou test, commonly known as the PAP test, whereby cells of the cervix are obtained by scraping the extocervix and endocervix with a collection device such as a spatula, brush, or cervical broom.

The sampled cells can be retrieved by smearing the specimen collection device onto a microscope slide and discarding the entire device. Most of the sampled cells are not transferred and discarded as waste together with the device. The transferred cells may be unrepresentative of a patient's actual condition.

Alternatively, a liquid-based method of transferring and storing sampled cells may be used. Instead of "smearing" cervical cells onto a slide, the portion of the collection device carrying the sampled cells is dipped into a preservative solution and swirled to release the sampled cells to the solution. The transferred cells are contained in the vial with the preservative solution and sent to a lab for analysis. This is more accurate than the traditional PAP smear. However, sample transfer depends on the time allowed and the force applied for transferring sampled cells to the preservative solution.

To avoid having to transport the entire cell collection device, the portion of the cell collection device carrying the cells can be separated from the remainder of the device. For instance, in case a wooden spatula is used, part of the sampling device can be broken off. This process requires the use of both hands to break the spatula or the use of a tool to cut off part of the spatula. This is tedious, cumbersome and unhygienic.

Alternatively, a cell collection device may be used with a shaft comprising a region thinner than the shaft. The cell carrying portion is then separated from the shaft by cutting or breaking the shaft in the thinner region. The exertion of a force on the shaft to break off the cell carrying portion is often excessive, causing a sampling pot containing the sample collection vial to tip over. As preservative solution is spilled, sampled cells are lost.

Sampling devices have been developed with a removable sampling head on a longitudinal handle. The head can be released from the longitudinal handle by tearing off the head from the longitudinal handle. That is, by applying lateral and opposing forces to obtain that longitudinal handle and sampling head move in opposite directions. These forces are applied by taking the longitudinal handle in one hand and removing the head of the shaft by using the other hand and applying a force moving the head way from the longitudinal handle. With this method the head risks to be contaminated when touched. Moreover sampled cells may transfer causing sample losses. In order not to contaminate the preservative solution, or cause liquid spills and consequently sample losses, the head needs to be removed from the longitudinal handle prior to transfer of the head to the vial. This is a tedious and messy process.

There remains a need in the art to further improve sampling devices, in particular to improved devices for obtaining a sample from a body cavity. It will be will be appreciated that there exists a need for sampling devices which allow easy and reliably transfer of sampled material and which are relatively durable, will not easily break and are available through a straightforward and cost effective manufacturing process.

It is accordingly one of the objects of the present invention to overcome or ameliorate at least one of the disadvantages of the prior art, or to provide a useful alternative.

### Summary of the invention

The inventor has found that concerns related to the transfer of sampled cells from a sampling device can be minimized by providing a novel and inventive sampling device.

In particular the present invention relates to a cell collection device, comprising a means for collecting cells and a longitudinal handle whereby said means for collecting cells is attachable to the longitudinal handle using a snap release joint. This has for advantage that a longitudinal handle and means for collecting cells can be easily separated with the use of a simple and single hand movement.

In an embodiment of the invention, a cell collection device as described above is provided, whereby said means comprises a distal end and a proximal end and said distal end is adapted to engage the means for collecting cells (2) in a snap-release relation. This has for advantage that only the part carrying the sampled cells is retrieved, minimizing the weight in transport to a lab.

In an embodiment of the invention, a cell collection device is provided, whereby the snap-release joint (4) comprises a dovetail (10) and a dovetailed slot (11); the dovetail is positioned at said distal end and is adapted to receive the dovetailed slot. This has for advantage that the manufacturing of the snap-release joint requires a minimum of tooling, contributing to an economic manufacturing cost of the sampling device.

In an embodiment of the invention, a cell collection device as described above is provided, whereby said means for collecting cells (2) is releasable from said longitudinal handle by means of a pressure force applied on the snap-release joint (4).

In an embodiment of the invention, a cell collection device as described above is provided, whereby the cell collection device (1) is a cervical brush. This has for advantage that the retrieval of cells sampled from the cervix is simplified, reliable and risk of sample contamination is minimized.

In an embodiment of the invention, a cell collection device as described above is provided, whereby said means for collecting cells (2) comprises a fortification zone (11). This has for advantage that the forces exerted on the snap-release joint are not dispersed and are concentrated on the joint. Damage to the means for collecting cells is reduced.

In an embodiment of the invention, a cell collection device as described above is provided, whereby said longitudinal handle (3) and/or said means for collecting cells are made from an elastic material. This has for an advantage that a force exerted on the longitudinal handle of a cell collection device is easily translated to the snap-release joint to release the means for collecting cells. The longitudinal handle acts as a lever.

In an embodiment of the invention, a cell collection device as described above is provided, whereby longitudinal handle (3) has an area with a means for providing grip (14). This has for advantage that additional grip is provided to the instrument. The force applied to disengage longitudinal handle and means for sampling cells can be decoupled more accurately.

In an embodiment of the invention, a cell collection device as described above is provided, obtainable by co-moulding. This has for advantage that the instrument is easily obtainable, with well-known manufacturing techniques, in a minimum of manufacturing steps, hence at affordable prices. This has for advantage that the construction is easy to manufacture. Furthermore it allows for exchangeable parts.

In another aspect of the invention means for collecting cells (2) are provided, comprising a fortification zone (11) and a shape suitable for being secured to a longitudinal handle (3) to form a snap-release joint (4). This has for advantage that an improved head portion is provided for a cell collection device, in particular the sampling device of the invention. The wall thickness of the material of the fortification zone and shape provides additional support. Risk of a torn connection between longitudinal handle and the means for collecting cells is reduced.

In another aspect of the invention a method of collecting a sample with a cell collection device is provided, comprising the steps of:
a) providing a cell collection device (1) comprising a means for collecting cells (2) and a longitudinal handle (3) mutually securable with a snap-release joint (4); and
b) applying a pressure force on the snap-release joint (4) suitable to disengage the longitudinal handle from the means for collecting a sample.

This has for effect that a simple and straightforward sampling technique with a minimum risk of contamination, is provided for the collection and retrieval of cells. It contributes to the reliability of sample collection methods. The method reduces the need for sample collection repeats.

In an embodiment of the invention a method as described above is provided, further comprising the step of:
c) positioning the means for collecting cells (2) against a surface (6) inside a sample collection vial (5).

This has for effect that the cell collection device can be transferred to a fixation fluid, prior to the disconnection of the sampling head from the longitudinal handle. Consequently, the transfer of sampled cells is maximized; the risk of loosing cells is reduced.

In an embodiment of the invention a method as described above is provided, whereby the pressure force provides an angle between the longitudinal axis of said longitudinal handle (3) and the longitudinal axis of said means for collecting cells.

In an embodiment of the invention a method as described above is provided, whereby the pressure force provides an angle of between 175° to 160° prior to release of said longitudinal handle from said means for collecting cells.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### Figure legend

The invention will hereafter be elucidated with reference to the drawings, which show embodiments of the cell collection device according to the invention by way of example.
**Figure 1** schematically illustrates a cell collection device 1 according to the invention comprising a means for collecting cells 2 in the form of a cervical brush, attached to a longitudinal handle 3 with a snap-release joint 4. The longitudinal handle is provided with a means for providing grip 14.
**Figures 2A and 2B** schematically illustrate a method according to the invention.
**Figure 3** schematically illustrates a cell collection device of the prior art with a snap-fit connection 8. This connection requires the use of lateral forces acting in opposite directions to obtain disengagement of the means for collecting cells from the longitudinal handle.
**Figure 4** schematically illustrates a cell collection device according to the invention with a snap-release connection 4. The snap-release connection comprises a dovetail 9 and dovetail slot 10. Also visible on figure 4 is a reinforcement zone 11.
**Figure 5** schematically illustrates a detail of a snap-release connection of the invention, showing a dovetail 9 whereby the width of the top region 12 and height 13 are about equal.

### Detailed description

Unless defined otherwise, all technical and scientific terms used herein have the same meaning as is commonly understood by one of skill in the art.

The articles "a" and "an" are used herein to refer to one or to more than one, i.e. to at least one of the grammatical object of the article.

Throughout this application, the term "about" is used to indicate that a value includes the standard deviation of error for the device or method being employed to determine the value.

The recitation of numerical ranges by endpoints includes all integer numbers and, where appropriate, fractions subsumed within that range (e.g. 1 to 5 can include 1, 2, 3, 4 when referring to, for example, degrees, and can also include 1.5, 2, 2.75 and 3.80, when referring to, for example, measurements). The recitation of end points also includes the end point values themselves (e.g. from 1.0 to 5.0 includes both 1.0 and 5.0).

The present invention relates to a tool for sampling cells, in particular human tissue cells, more in particular cells collected from a body cavity such as the cervix. The sampling tool or cell collection device according to the invention comprises a longitudinal handle, a portion equipped for the collection of cells and a snap-release joint.

With the term "snap-release joint" as used herein, is meant a mechanical decoupling system where part-to-part attachment accomplished with locating and locking features that are homogenous with one or the other of the components when joined. Decoupling requires the flexible locking features to move aside for disengagement with the mating part, followed by return of the locking feature toward its original position. The joint is executed so that a locking feature is releasable by the application of a pressure force on the joint.

A snap-release type of joint has for advantage that it requires no additional pieces, materials or tools to carry out the attaching function. Snap-releases eliminate screws, clips, adhesives, or other joining methods. They provide simplified assemblies and associated costs.

The snap-release joint of the invention is of a size allowing separation of the joined parts that is the means for collecting cells and the longitudinal handle, when applying pressure on the snap-release joint. This pressure can be obtained by flexing the longitudinal axis of the handle towards the longitudinal axis of the means for collecting cells.

By the term "flexing" as used herein, is meant, the act or an instance of flexing; bending, reducing the angle between two axes.

By the term "flexing force" as used herein, is meant the application of a force that bends a joint; especially a joint between a longitudinal handle and a means for collecting cells, so that the angle between them is decreased.

In an embodiment of a method of the invention, the pressure force and/or flexing force provides an angle between the longitudinal axis of said longitudinal handle (3) and the longitudinal axis of said means for collecting cells.

In an embodiment of a method of the invention, the pressure force and/or flexing force provides an angle of between 175° to 160° prior to release of said longitudinal handle from said means for collecting cells. In a preferred embodiment of a method of the invention the pressure force and/or flexing force provides an angle of at least 175°, at least 170°, at least 165°, or at least 160° prior to release of said longitudinal handle from said means for collecting cells.

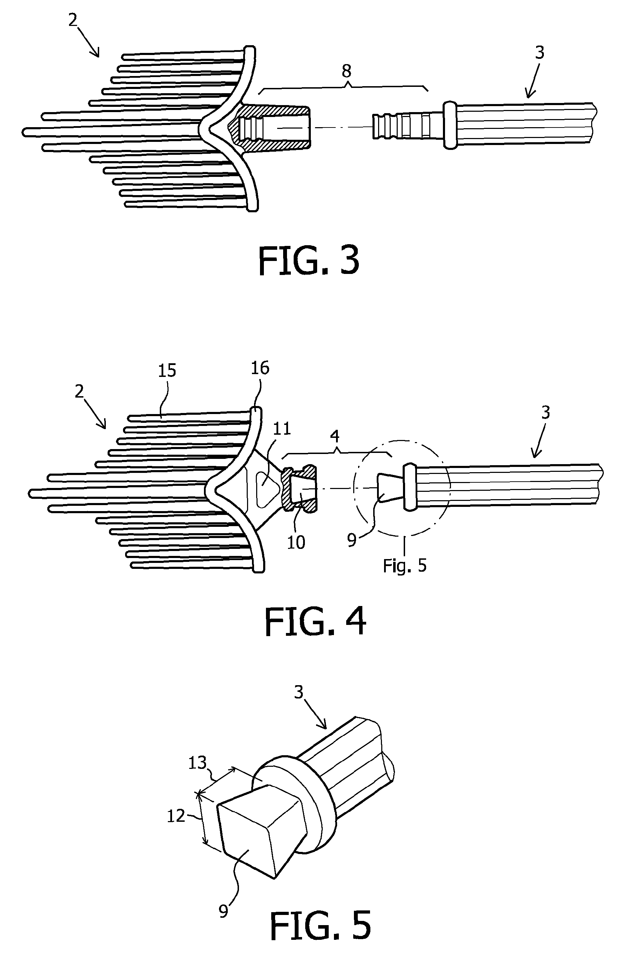

Known in the art are cell collecting devices with a snap-fit connection, for instance such as depicted in **Figure 3**. The longitudinal handle and the means for collecting cells are joined together by a snap-fit connection. Separation of the longitudinal handle or shaft and the means for collecting cells can then be achieved by the application of lateral forces moving in opposite direction. The means for collecting cells is grasped with one hand and the longitudinal handle is grasped with a second hand. Both these parts are then moved in opposite direction to obtain detachment. In order to avoid that the hand is positioned over the area comprising sampled tissue cells, the carrier 16 can be V-shaped. This provides support for tearing off the means for collecting cells from the longitudinal handle.

The inventors realized that this device could be improved by providing a connection of a different type, in particular, one in which a means for collecting cells and a longitudinal handle can be releasably connected and can be released upon flexing of the longitudinal handle towards the means for collecting cells.

By the term "snap-release joint" it is meant a first and second part that mate together to form a snap-release connection. The first part is snapped out and released from the second part by the application of a flexing force on the joint. The inventors have named this type of connection a snap-fit joint.

In an embodiment of the invention the first part of the snap-release connection is positioned at one extreme end of the longitudinal handle. It is adapted to receive a counterpart which is provided to receive the first part and provide a form matching connection. Preferably, the counterpart is attached to a fortification zone.

Preferably one part is a dovetail; another part is a dovetailed slot. The dovetail and dovetailed slot together form a snap-release joint.

The configuration of a part as dovetail has the advantage that it is easy to make in a manufacturing process. The presence of rounded corners reduces the requirement for additional tooling to make edges and corners in a part well defined and fitting for their counterpart. A dovetail structure is robust.

Preferably the dovetailed slot is positioned at one extreme end of the means for collecting cells. Consequently, when snapped-in to the longitudinal handle, the cell collection means is attached to the longitudinal handle and they can function together as part of the whole cell collection device.

In order for a snap-release joint to be releasable in this way, its dimensions are preferably of a small size. In an embodiment of the invention, the width of the first part of the snap-release joint is between 2-6 mm and the height is between 2-6 mm. In an embodiment of the invention, the width of the first part of the snap-release joint about equals its height. In a preferred embodiment of the invention, the width of the first part of the snap-release joint is about 3 mm and the height is about 3 mm.

Preferably the snap-release joint is positioned in a cell collection device where the means for collecting cells ends and the longitudinal handle begins. That is, the snap-release connection is provided such that an extreme end of the longitudinal handle is joined to an extreme end of the means for collecting cells. This has for advantage that a large area of the part which is kept for further processing comprises sampled tissue cells. Upon transfer to a collection vial with liquid, their will be less space that is covered with liquid without cells being present. This reduces solvent consumption and hence costs. It can also reduce the weight of the samples for transfer to a lab, further reducing costs.

In an embodiment of the invention, the longitudinal handle of the cell collection device as described above has an elastic construction. Preferably the longitudinal handle of the cell collection device is made from an elastic material, for instance plastic, such that bending the longitudinal handle or longitudinal handle does not result in a breakage of the material. This provides safety to the device. Additionally it has for effect that upon bending the longitudinal handle a force is also translated further down the longitudinal handle onto the snap-release joint. With a force sufficient to move the one part out of the receiving counter-part of the snap-release joint, the parts comprising the snap-release connection will disconnect. A separate longitudinal handle and means for cell collection are the result. The longitudinal handle can leverage that the parts of the snap-release joint are moved apart and the connection is released.

In an embodiment of the invention, the longitudinal handle of the cell collection device has a portion including one or more means for providing grip (14). These grip enhancing means can be structures protruding from the surface of the longitudinal handle, such as for instance ribs, edges, or buds. Alternatively, the surface of a longitudinal handle can comprise holes, so that the surface is made less smooth and easier to grip. Such structures can easily be obtained by manufacturing techniques such as injection moulding or extrusion. Longitudinal handles with a means for improved gripping provide enhanced dexterity. A longitudinal handle and hence cell collection device can be manipulated and positioned more accurately.

The cell collection device of the invention is obtainable by well-known manufacturing techniques. Suitable manufacturing techniques can be injection moulding and extrusion. By the term "injection moulding" as used herein a manufacturing technique is meant for making parts from thermoplastic and thermosetting plastic materials in production. Molten plastic is injected at high pressure into a mould, which is the inverse of the product's shape. After a product is designed, moulds are made from for instance metal, usually either steel or aluminium, and precision-machined to form the features of the desired part.

Preferably the cell collection device is obtained by co-moulding. The application of co-moulding to manufacture the device of the invention has the advantage that the assembly of the parts comprising the cell collection device is done in the mould. Co-moulding or in-mould assembly has the advantage that it brings a downstream action into the molding process, hence eliminating secondary operations. Or in other words, components which would otherwise be produced separately and then put together are now produced in the mould. The use of co-moulding can provide a more consistent product that is not subject to the same warpage or shrinkage issues seen when consecutive operations are employed, especially when two or more portions of a part are to be mated. The co-moulding technique can avoid the need for separate presses and moulds, reducing labour and floorspace requirements.

The in-mould assembly can be obtained by multiple shots. Preferably the longitudinal handle provided with a shape at its extremity for forming a snap-release joint is moulded in a first moulding step. Preferably the means for collecting cells is moulded over the shape to form a snap-release joint. In this way the hardest material is formed first, followed by a covering of softer material.

The longitudinal handle and/or means for collecting cells can be made from an elastic material or a rigid material. In a preferred embodiment of the invention the longitudinal handle and/or means for collecting cells are made from an elastic material.

By the term "elastic material' as used herein it is meant, a material that is flexible and/or bendable. By the term "rigid material' as used herein it is meant, a material that is stiff.

In a preferred embodiment of the invention the longitudinal handle of the cell collection device is manufactured from a material selected from the group comprising polypropylene, acrylonitrille-butadiene-styrene polymer or polyamide.

In a preferred embodiment of the invention the sample collection means is manufactured from a material selected from the group comprising low-density polyethylene, high-density polyethylene or ethylene vinyl acetate polymer.

A cell collection device obtained with these materials provides an integrally connected device yet with movable and detachable parts.

In an embodiment of the invention, the cell collection device is a device for obtaining a sample from a body cavity, in particular the uterus mouth or cervix.

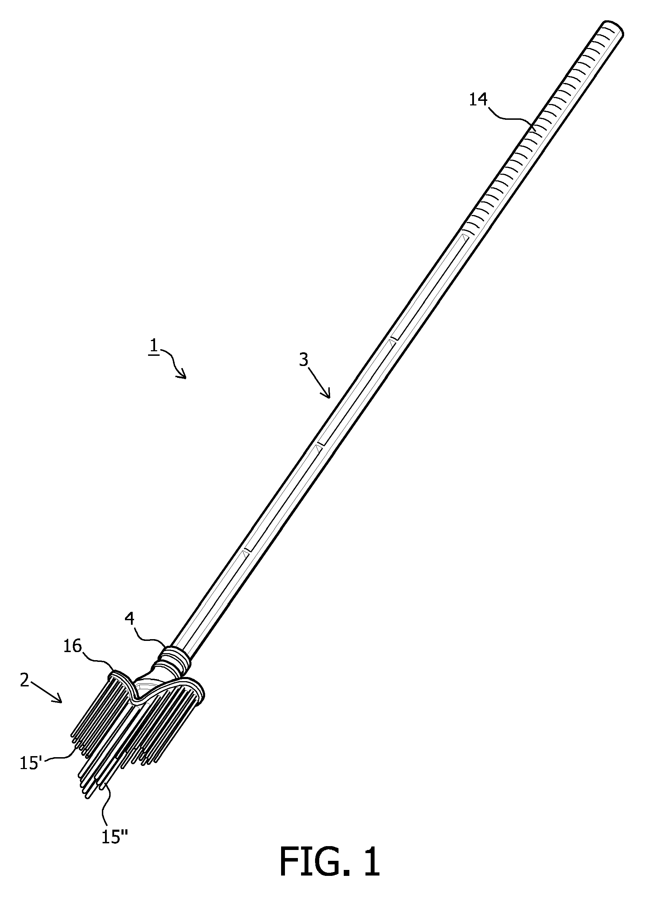

In an embodiment of the invention, the cell collection device as described above is a cervical brush. In an embodiment of the invention, the cell collection device is as depicted in Figure 1. This device comprises a longitudinal handle 3 having a circular transverse section, one end thereof being provided with a means for sampling cells 2, which caries a plurality of bristles 15 extending parallel to the longitudinal handle 3, so that a brush is formed. The bristles are of different types: the bristles 15' located outwardly of the transverse centre of the carrier 16 are somewhat smaller than the bristles 15" provided in the longitudinal plane in the centre of the carrier.

In a further aspect, the invention relates to a method of collecting a sample with a cell collection device of the invention. In a method of the invention a cell collection device is used whereby the longitudinal handle is removably attached to the means for collecting cells by means release joint. In a preferred embodiment of a method of the invention, the means for collecting cells is positioned against a surface. This surface is preferably the bottom surface of a sample collection vial so that upon detachment of the sampling portion, a transfer is no longer required. The sample collection vial can already be filled with sample fluid. The movement the means for collecting cells makes by snapping out of the joint can contribute to the detachment and collection of sampled cells.

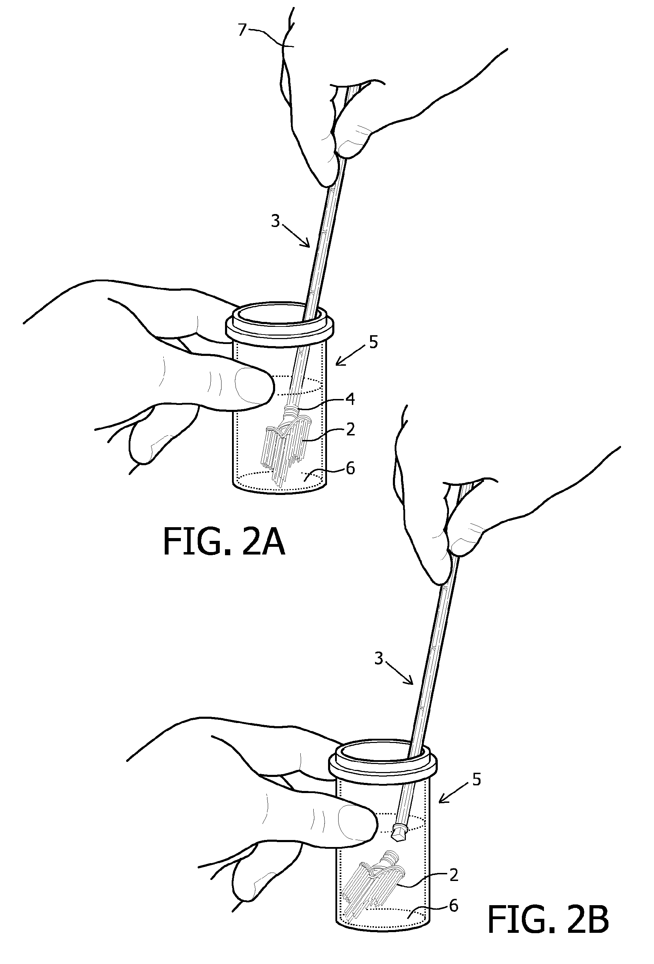

In a preferred embodiment, a method of the invention is as depicted in **Figures 2A** and **2B****.** A sampling device of the invention is positioned inside a sample collection vial 5. The means for collecting cells 2 is detached from the longitudinal handle 3 by positioning the sample brush against the bottom surface 6 of the vial and applying a slight pressure on the longitudinal handle with one hand 7. The means for collecting cells detaches from the longitudinal handle (Figure 2B). The longitudinal handle is removed and discarded. The sampling vial is filled with liquid prior to or after the head and longitudinal handle are disengaged. The vial is closed and sent to a lab for further processing.

In a further aspect, the invention relates to a means for collecting cells comprising a fortification zone and a shape suitable for being secured to a longitudinal handle by means of a snap-release joint. In a prefered embodiment of the invention, means for collecting cells comprising a fortification zone and a shape suitable for being secured to a longitudinal handle by means of a snap-release joint are as depicted in **Figure 4**. The head of a cervical brush 2 is displayed. At the end opposite the central bristles a portion 10 is positioned. This portion is in the shape a dovetail slot 10. Together with a form matching portion 9, the portion 10 is capable of forming a snap-release fit. Attached to the dovetail slot is a fortification zone 11. The fortification zone provides support to the carrier of the bristles 15. It also serves to position the means for collecting cells against a surface. In **Figure 5** a detailed representation of the form matching portion 9 is displayed. The width 12 of the form matching portion 9 is about equal to the height 13 of the form matching portion.

## Claims

1. Cell collection device (1), comprising
a) a means for collecting cells (2) and
b) a longitudinal handle (3)
whereby said means for collecting cells (2) is attachable to the longitudinal handle (3) using a snap release joint (4).

2. Cell collection device according to claim 1, whereby said means comprises a distal end and a proximal end and said distal end is adapted to engage the means for collecting cells (2) in a snap-release relation.

3. Cell collection device according to claim 2, whereby the snap-release joint (4) comprises a dovetail (10) and a dovetailed slot (11); the dovetail is positioned at said distal end and is adapted to receive the dovetailed slot.

4. Cell collection device according to any of the above claims, whereby said means for collecting cells (2) is releasable from said longitudinal handle by means of a pressure force applied on the snap-release joint (4).

5. Cell collection device according any of the above claims, whereby the cell collection device (1) is a cervical brush.

6. Cell collection device according to any of the above claims, whereby said means for collecting cells (2) comprises a fortification zone (11).

7. Cell collection device according to any of the above claims, wherein said longitudinal handle (3) and/or said means for collecting cells are made from an elastic material.

8. Cell collection device according to any of the above claims, wherein said longitudinal handle (3) has an area with a means for providing grip (14).

9. Cell collection device according to any of the above claims, obtainable by co-moulding.

10. Means for collecting cells (2), comprising a fortification zone (11) and a shape suitable for being secured to a longitudinal handle (3) to form a snap-release joint (4).

11. Method of collecting a sample with a cell collection device, comprising the steps of:
a) providing a cell collection device (1) comprising a means for collecting cells (2) and a longitudinal handle (3) mutually securable with a snap-release joint (4); and
b) applying a pressure force on the snap-release joint (4) suitable to disengage the longitudinal handle from the means for collecting a sample.

12. Method according to claim 11, further comprising the step of :
c) positioning the means for collecting cells (2) against a surface (6) inside a sample collection vial (5).

13. Method according to claim 11 or 12 whereby the pressure force provides an angle between the longitudinal axis of said longitudinal handle (3) and the longitudinal axis of said means for collecting cells.

14. Method according to claim 13 whereby the pressure force provides an angle of between 175° to 160° prior to release of said longitudinal handle from said means for collecting cells.
